# EUROPÄISCHE PATENTSCHRIFT

(11) **EP 2 818 227 B1**
(45) Veröffentlichungstag und Bekanntmachung des Hinweises auf die Patenterteilung: **17.08.2016**
(21) Anmeldenummer: 14168734.3
(22) Anmeldetag: 16.05.2014
(51) Int. Cl.: B01D 53/62, C10L 3/08, C07C 31/04

(54) **Verfahren zum Betreiben eines gebrannten Kalk, Dolomit, Magnesia oder Zement erzeugenden Schachtofens sowie Schachtofen-Werksanlage zur Erzeugung von gebranntem Kalk, Dolomit, Magnesia oder Zement**
Method for operating a shaft furnace for the production of burnt lime, dolomite, magnesia or cement and shaft furnace plant installation for producing fired lime, dolomite, magnesia or cement
Procédé d'opération d'un four droit produisant de la chaux vive, de la dolomite, de la magnésie ou du ciment et installation de four droit destinée à la production de chaux vive, de dolomite, de magnésie ou de ciment

(30) Priorität: 28.06.2013 DE 102013010909
(43) Veröffentlichungstag der Anmeldung: 31.12.2014
(73) Patentinhaber: Fels-Werke GmbH, 38640 Goslar (DE)
(72) Erfinder: Stumpf, Thomas Dr., 38667 Bad Harzburg (DE); Boenkendorf, Ulf Dr., 31188 Holle (DE); Moeller, Roland, 38667 Bad Harzburg (DE)
(74) Vertreter: Patent- und Rechtsanwälte Dr. Solf & Zapf

(56) Entgegenhaltungen:
- EP-A1- 2 100 869
- FR-A1- 2 977 089

## Beschreibung

Die Erfindung betrifft ein Verfahren zum Betreiben eines gebrannten Kalk, Dolomit, Magnesia oder Zement erzeugenden Schachtofens sowie eine Schachtofen-Werksanlage zur Erzeugung von gebranntem Kalk, Dolomit, Magnesia oder Zement mit einem Schachtofen.

Gebrannter Kalk, Dolomit, Magnesia und Zement werden in der Regel hauptsächlich aus natürlichen Rohstoffen wie Kalkgestein und/oder Kalkgesteinsmaterial, z. B. in Form von Waschrückständen von Kalkgesteinen, und/oder Tonschiefermaterial und/oder Tonmaterial und/oder Mergel und/oder Dolomitgestein und/oder Magnesit hauptsächlich in Schachtöfen erzeugt.

Die Calcination der natürlichen Rohstoffe wie Karbonatgesteine in z. B. Kalk- und Zementwerken mit Schachtöfen erfordert einen sehr hohen Energiebedarf. Zumeist werden fossile Brennstoffe verwendet, die CO₂ als Koppelprodukt aus der thermischen Zersetzung der Rohstoffe erzeugen. Die folgende Tabelle zeigt beispielhaft die Hauptbestandteile der Abgase eines Kalkschachtofens nach dem Stand der Technik.

| Typische Werte | | Kalkschachtofen NSO |
|---|---|---|
| | | |
| N₂ | Vol-% | 53 |
| O₂ | Vol-% | 0,2 |
| CO | Vol-% | 2 |
| CO₂ | Vol-% | 17 - 30 |
| H₂O | Vol-% | 1,2 - 7,5 |

Es sind Verfahren und Anlagen zur Erzeugung von gebranntem Kalk oder Zement bekannt, mit denen der Energieaufwand und die CO₂-Emission bei der Erzeugung von gebranntem Kalk oder Zement in Schachtöfen reduziert werden kann. Zum Beispiel wird Energie zurückgewonnen durch optimierte Verbrennungsführung oder durch besondere Schachtofenkonstruktionen mit mindestens zwei Schächten, in denen die Abwärme aus einer Brennzone eines Ofenschachtes eines sogenannten Parallelschachtofens in den anderen Ofenschacht zur Vorwärmung des Brenngutes eingeleitet wird. (Zement-Kalk-Gips, Nr. 3/1973, Seiten 119 bis 123; ZKG International, Nr. 6/1995 (48. Jahrgang), Seiten 312 bis 322; DE-OS 2927819; EP 1038851 A1)

Des Weiteren ist in diesem Zusammenhang bekannt, ein Brennstoffgemisch zu verwenden, das mit technischen Sauerstoff angereicherte Luft enthält, wobei zudem das CO₂-haltige Abgas des Schachtofens als Verdünnungsgas in das Brennstoffgemisch eingeführt und/oder als Kühlgas in eine Kühlzone des Schachtofens eingeleitet wird (DE 10 2010 019 330 A1).

Außerdem ist ein Verfahren zur Herstellung von Methanol durch Verwendung von Kohlenstoffdioxid aus dem Abgas fossil befeuerter Kraftwerke, Heizkraftwerke und anderer Emittenten wie z. B. aus Zementwerken der Zementindustrie, aus chemischen Anlagen, aus Stahlwerken, aus Biogas- und/oder Biomasseanlagen od. dgl. bekannt, wobei Kohlenstoffdioxid aus dem Abgas z. B. der fossil befeuerten Kraftwerke über eine Methanolsynthese unter Verwendung von Wasserstoff und Katalysatoren in Methanol umgesetzt wird. Dabei wird Methanol unter Verwendung von Wasserstoff hergestellt, der durch Elektrolyse von Wasser mit Hilfe von elektrischem, aus regenerativen Energiequellen stammendem Strom gewonnen wurde. Vorgeschlagen wird, überschüssige Elektroenergie aus erneuerbaren Energien für die Elektrolyse zu verwenden und den bei der Elektrolyse entstehenden überschüssig zur Verfügung stehenden Sauerstoff für den Verbrennungsprozess der fossil befeuerten Kraftwerke einzusetzen (DE 10 2009 007 567 A1).

Des Weiteren ist aus der EP 2 100 869 A1 ein Verfahren zur Herstellung von Methanol durch die Verwertung von Kohlendioxid aus dem Abgas fossil befeuerter Kraftwerke und anderer Emittenten bekannt. Dabei wird Kohlendioxid aus dem Abgas der fossil befeuerten Kraftwerke für eine Methanolsynthese mit regenerativ gewonnenem Wasserstoff vermischt und unter Verwendung von Katalysatoren in Methanol umgesetzt.

Aufgabe der Erfindung ist, eine Schachtofenanlage zum Erzeugen von gebranntem Kalk, Dolomit, Magnesia oder Zement so zu betreiben, dass der CO₂-Ausstoß gegen Null gefahren werden kann.

Diese Aufgabe wird durch die Merkmale des Anspruchs 1 und 6 gelöst. Vorteilhafte Weiterbildungen der Erfindung werden in den von diesen Ansprüchen abhängigen Ansprüchen gekennzeichnet.

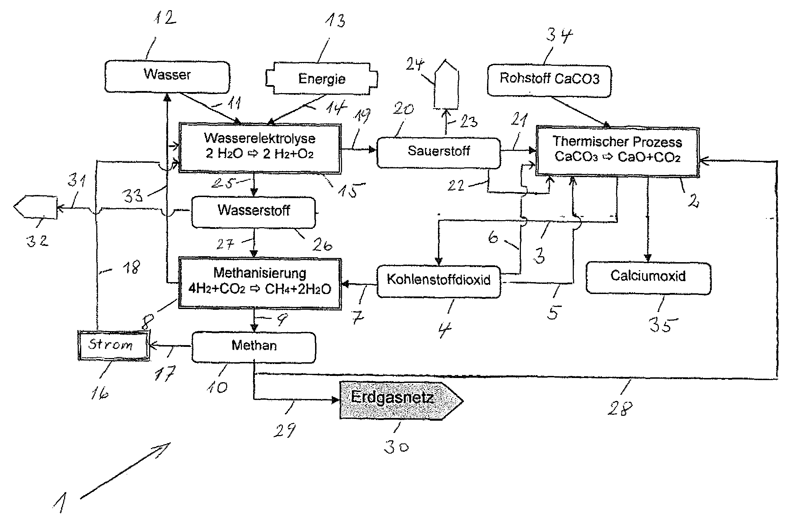

Anhand der Zeichnung wird die Erfindung im Folgenden beispielhaft näher erläutert.

Nach der Erfindung wird in einer Schachtofenanlage 1 z. B. zum Kalk- oder Dolomit- oder Magnesia- oder Zementbrennen in einem zumindest eine obere, Rohstoff z. B. aus einer Rohstoffhalde 34 aufnehmende Rohstoff-Einlaufzone, eine mittlere Brennzone und eine untere Brenngut-Auslaufzone aufweisenden Schachtofen 2 mit einer kontrollierten Ofenführung ein gefiltertes Abgas aus dem Schachtofen 2 aufgefangen und über mindestens eine Abgasleitung 3 in mindestens einen Abgasspeicher 4 geleitet und gespeichert; das Abgas setzt sich z. B. hauptsächlich wie folgt zusammen:

| | |
|---|---|
| H₂O_{Dampf}: | 1 bis 7, insbesondere 2 bis 5 Vol.-% |
| CO₂: | 60 bis 99, insbesondere 80 bis 98 Vol.-% |
| Rest jeweils verunreinigende Gase | |

Am unteren Ende der Auslaufzone des Schachtofens 2 wird das Brenngut, z. B. der gebrannte Kalk oder Zement oder Dolomit oder Magnesia, einem Bunker 35 zugeführt. Der Begriff "Schachtofen" umfasst im Rahmen der Erfindung alle bekannten Schachtofentypen zur Erzeugung der oben genannten gebrannten Produkte.

Aus dem Abgasspeicher 4 wird zweckmäßigerweise mindestens ein Kühlabgasstrom über mindestens eine Kühlabgasstromleitung 5 abgezweigt und zum Kühlen in eine Kühlzone des Schachtofens 2 geleitet. Zudem wird vorzugsweise mindestens ein Verdünnungsabgasstrom über mindestens eine Verdünnungsabgasstromleitung 6 abgezweigt und z. B. in einer Brennstoffgemischanlage des Schachtofens 2 zum Verdünnen des Brennstoffgemisches verwendet oder anderweitig dem Brennstoffgemisch zugeführt. Das Kühlabgas und/oder das Verdünnungsgas werden somit in die Ofenatmosphäre integriert.

Zumindest eine Teilmenge, vorzugsweise die gesamte Menge des restlichen überschüssigen Abgases wird über eine Abgasleitung 7 in mindestens eine an sich bekannte, der Schachtofenanlage 1 beigeordnete Methan- oder Methanol-Reaktoranlage 8 geleitet und dort in an sich bekannter Weise im Gemisch mit Wasserstoff Methan oder Methanol erzeugt. Das Methan oder Methanol wird über mindestens eine Gasleitung oder Flüssigkeitsleitung 9 in mindestens einen Methan- bzw. Methanolspeicher 10 eingeleitet.

Der Wasserstoff wird in mindestens einer Elektrolyseanlage 15 mit einem an sich bekannten Elektrolyseprozess durch Spaltung von Wasser in Wasserstoff und Sauerstoff gewonnen. Erfindungsgemäß erfolgt die Erzeugung von Wasserstoff und Sauerstoff aus Wasser unter Verwendung von dem Methanisierungs- oder Methanolisierungsreaktor (8) entnommenem Wasser. Nach einer Ausführungsform der Erfindung wird für die Elektrolyse zusätzlich mit mindestens einer Wasserleitung 11 aus dem öffentlichen Wasserversorgungsnetz 12 entnommenes Wasser verwendet.

Erfindungsgemäß wird für den Elektrolyseprozess werksanlageneigenständig erzeugter elektrischer Strom verwendet, der mit mindestens einem in die erfindungsgemäße Werksanlage 1 integrierten, werkseigenen Stromerzeugungsaggregat 16 erzeugt wird, das ein Antriebsmittel, z. B. einen Brennstoff-Motor oder eine Brennstoff-Turbine, aufweist, das mit dem aus dem erfindungsgemäßen Verfahren stammenden Methan und/oder Methanol betrieben wird. Solche Stromerzeugungsanlagen sind z. B. Dieselaggregate. Dazu wird zweckmäßigerweise z. B. über mindestens eine Gas- bzw. Flüssigkeitsleitung 17 Methan oder Methanol dem Stromerzeugungsaggregat 16 zugeführt.

Die Stromversorgung des Elektrolyseprozesses in der Elektrolyseanlage 15 kann zweckmäßigerweise auch mittels kombinierter Stromeinspeisung aus dem öffentlichen Stromnetz 13 und dem Stromerzeugungsaggregat 16 erfolgen, wobei der elektrische Strom des Stromerzeugungsaggregats 16 über mindestens eine Stromleitung 18 der Elektrolyseanlage 15 zugeführt wird.

Der Elektrolyseprozess wird nach einer Ausführungsform der Erfindung zusätzlich mit elektrischem Strom bzw. mit elektrischer Energie, z. B. aus dem öffentlichen Stromnetz 13, insbesondere mit Strom aus erneuerbaren Energien betrieben, und zwar insbesondere mit Strom, der aus sogenannten Energieerzeugungsspitzen (sogenanntem Überschussstrom) stammt, der relativ preiswert erhältlich ist.

Der elektrische Strom wird über mindestens eine Stromleitung 14 der Elektrolyseanlage 15 zugeführt.

Im Elektrolyseprozess gewonnener Sauerstoff wird über mindestens eine Gasleitung 19 in mindestens einen Sauerstoffspeicher 20 eingespeist und zumindest mit einer Teilmenge über mindestens eine Gasleitung 21 dem Schachtofen 2, zweckmäßigerweise mindestens einer Brennstoffgemischanlage des Schachtofens 2 zugeführt und/oder über mindestens eine Gasleitung 22 dem aus dem Abgasspeicher 4 stammenden Kühlgas des Schachtofens 2 beigegeben und somit zur Verbrennung des Brennstoffs, der zur Calcinierung des zu brennenden Rohstoffs zur Erzeugung des gebrannten Kalkes oder Zements oder Dolomits oder Magnesia verwendet wird, eingesetzt. Soweit Sauerstoff im Überschuss erzeugt wird, wird er z. B. an Dritte 24 über mindestens eine Gasleitung 23 abgegeben. Wenn nicht ausreichend Sauerstoff aus der Elektrolyse zur Verfügung steht, kann zusätzlich z. B. von Dritten erworbener Sauerstoff verwendet werden.

Im Elektrolyseprozess gewonnener Wasserstoff wird über mindestens eine Gasleitung 25 in mindestens einen Wasserstoffspeicher 26 eingespeist und zumindest mit einer Teilmenge über mindestens eine Gasleitung 27 der Reaktoranlage 8 zugeführt, wo er mit zugeführtem Überschussabgas aus dem Abgasspeicher 4 zur Reaktion gebracht, woraus die Erzeugung in an sich bekannter Weise von Methan und/oder Methanol resultiert. Überschüssiger Wasserstoff kann z. B. an Dritte 32 über mindestens eine Gasleitung 31 abgegeben werden.

Das erzeugte Methan oder Methanol wird, wie bereits beschrieben, in den Speicher 10 eingespeist und zumindest mit einer Teilmenge über mindestens eine Gas- bzw. Flüssigkeitsleitung 28 dem Schachtofen 2 als Brennstoff, z. B. der Brennstoffgemischanlage des Schachtofens 2 zur Bildung eines Brennstoffgemisches zugeführt. Eine weitere Teilmenge wird, wie bereits beschrieben, für das Betreiben des Stromerzeugungsaggregats 16 verwendet. Verbleibende Restmengen werden z. B. über mindestens eine Gas- bzw. Flüssigkeitsleitung 29 z. B. an Dritte 30 z. B. ein Erdgasnetz abgegeben oder anderweitig verwendet.

Bei der Herstellung des Methans oder Methanols anfallendes Wasser wird zweckmäßigerweise z. B. über mindestens eine Wasserleitung 33 aus der Reaktoranlage 8 der Elektrolyseanlage 15 zugeführt.

Das erfindungsgemäße Verfahren und die erfindungsgemäße Werksanlage 1 z. B. zum Brennen von Kalk oder Zement oder Dolomit oder Magnesia können so ausgelegt und betrieben werden, dass das gesamte überschüssige Abgas des Schachtofens 2 zu Methan oder Methanol verarbeitet wird und das ggf. überschüssige Methan und/oder Methanol an Dritte gewinnbringend abgegeben wird. Somit wird im Rahmen der Erfindung lediglich elektrische Energie und Wasser verbraucht, wobei im Falle von ausreichend werksanlagenseitig, eigenständig erzeugter elektrischer Energie und deren Verwendung im mit dem erfindungsgemäß gewonnenen Methan oder Methanol betriebenen Stromerzeugungsaggregat sogar nur noch Wasser verbraucht wird.

Die Erfindung zeigt in einer besonderen Ausführungsform somit einen neuen und nicht vorgegebenen Weg für den Betrieb industrieller thermischer Calcinierungsprozesse auf, die durch Verwendung von lediglich Sauerstoff und Kohlenstoffdioxid neben dem Brennstoff im Brennstoffgemisch ein hochkonzentriertes Kohlenstoffdioxid-Abgas erzeugen, das mittels Elektrolyse und Methanisierung zur Synthese des Erdgas-Substituts Methan oder Methanol verwendet wird und zudem verwendet wird, CO₂ des Abgases beim Betreiben des Schachtofens wiederzuverwenden, sowie den Sauerstoff der Elektrolyse im Brennstoffgemisch zu verwenden, das das eigens selbst hergestellte Methan oder Methanol enthält.

Die erfindungsgemäß verwendeten Prozessschritte sind im Einzelnen:

### Elektrolyse

Der chemische Prozess der Redoxreaktion "Wasserelektrolyse" läuft über folgende Reaktion ab:

| | |
|---|---|
| 2 H₂O (I) → 2H₂ (g) + O₂ (g) | ΔH_{R}⁰ = + 572 kJ |

Die elektrolytische Herstellung von Wasserstoff ist verfahrenstechnisch das einfachste und - bezogen auf die eingesetzte elektrische Energie - ein sehr effizientes Verfahren.

Die "klassische" alkalische Wasserelektrolyse mit einem Wirkungsgrad von etwa 70% (bezogen auf den oberen Heizwert des Wasserstoffs) für die Herstellung von Wasserstoff wird seit vielen Jahren dort eingesetzt, wo entweder elektrischer Strom günstig verfügbar ist, oder kleinere Mengen reinen Wasserstoffs für chemische Verfahren benötigt werden. Verschiedene neue Konzepte wurden in den letzten Jahren untersucht und verfahrenstechnisch entwickelt, um die Effizienz der alkalischen Wasserelektrolyse zu erhöhen. Wirkungsgrade über 80% sind technisch realisierbar. Da die Zellspannung von Elektrolyseuren und damit auch der Wirkungsgrad unmittelbar von der Stromdichte und damit der Leistung des Elektrolyseurs abhängen, nimmt die Effizienz von Elektrolyseuren im Teillastbereich zu. Dieses günstige Teillastverhalten ist besonders vorteilhaft für die erfindungsgemäße Kopplung von Elektrolyseuren mit variablem Stromangebot aus Windkraftanlagen oder Solarkraftwerken oder für das Lastmanagement mit Wasserstoffspeicherung in Netzen mit ausgeprägt wechselnden Lastprofilen. Diese Methode der Erzeugung von Wasserstoff ist bekannt und braucht deshalb auch nicht weitergehend erläutert zu werden.

### Methanisierung

Der chemische Prozess der "Methanisierung" läuft über folgende Reaktion ab:

| | |
|---|---|
| 3 H₂ + CO → CH₄ + H₂O (g) | ΔH_{R}⁰ =-206 kJ/mol |
| 4 H₂ + CO₂ → CH₄ + 2 H₂O (g) | ΔH_{R}⁰ =-165 kJ/mol |

Derzeit wird die Methanisierung nicht zur großtechnischen Gewinnung von Methan genutzt, da Methan billiger aus Erdgas gewonnen werden kann. Eine technische Anwendung der Methanisierung wird mit der Zunahme von Wind- und Solarenergie von zunehmender Bedeutung. Grundsätzlich erfolgt die Methanisierung mit Katalysatoren und wird in Festbett- und Wirbelschichtverfahren unterteilt. Die Reaktion bzw. der Prozess selbst ist exotherm. Die freiwerdende Wärme kann erfindungsgemäß sinnvoll genutzt werden, beispielsweise kann sie den Rohstoffen vor der Calcinierung für deren Trocknung außerhalb des Schachtofens zugeführt werden.

### Industrieller thermischer Prozess - Calcinierung

Das erfindungsgemäße Verfahren ist grundsätzlich für alle thermischen Prozesse geeignet, in denen Wärme aus fossilen oder regenerativen Brennstoffen verwendet wird. Idealerweise sind dies Prozesse mit einem hohen Energiebedarf wie z. B. das Brennen von Kalk, Dolomit, Magnesia oder Zement. Im Folgenden wird exemplarisch das Verfahren zum Brennen von Kalk (Calcinierung) beschrieben:
Der chemische Prozess der "Calcinierung" läuft über folgende Reaktion ab:

| | |
|---|---|
| CaCO₃ → CaO + CO₂ (g) | Δ H_{R}⁰ = + 178 kJ/mol |

Es handelt sich somit um einen stark endothermen Prozess. Zusätzlich ist beim Kalkbrennen die vergleichsweise hohe Menge an emittiertem CO₂ zu bewältigen. Die CO₂-Menge resultiert einerseits aus den Verbrennungsprodukten der bisher überwiegend eingesetzten fossilen Brennstoffe sowie andererseits aus der thermischen Zersetzung des Carbonatrohstoffs. Nachteilig an den herkömmlichen Calcinierprozessen ist, dass diese aufgrund des Betriebes mit atmosphärischer Luft zur Kühlung bzw. als Quelle des zur Verbrennung notwendigen Sauerstoffs einen hohen Stickstoffanteil im Abgas bzw. für eine spätere Nutzung einen vergleichsweise geringen Anteil von z. B. 30 Vol-% CO₂ im Abgas haben. Insofern wird im Rahmen der vorliegenden Erfindung ein Verfahren zum Betrieb von Calcinieröfen aufgezeigt, in dem einerseits der luftstämmige Sauerstoffanteil für die Oxidation ersetzt wird und andererseits Kohlenstoffdioxid des Abgases zumindest teilweise als Kühlgas verwendet wird. Kombiniert werden erfindungsgemäß diese Verfahrensschritte mit der Wasserelektrolyse sowie der Methanisierung von CO₂, wobei bei der Wasserelektrolyse freiwerdender Sauerstoff effektiv für die Verbrennung im Calcinierofen genutzt wird, bei der Calcinierung freiwerdendes und im Brenngasgemisch vorhandenes Kohlendioxid für die Methanisierung verwendet wird und durch die Verwendung von Elektrolysesauerstoff und Kohlenstoffdioxid bei der Calcinierung ein nahezu ausschließlich CO₂ enthaltendes Abgas erzeugt wird, das sich wegen der Reinheit insbesondere auch für die Methanisierung eignet.

Soweit vorangehend oder nachfolgend von Methan oder Methanisierung die Rede ist, ist gleichermaßen die Herstellung von Methanol und Methanolisierung gemeint.

Durch die Kombination der drei genannten Prozessschritte Elektrolyse, Methanisierung und die Brennstoff-Sauerstoff-Kohlenstoffdioxid betriebene Calcinierung ergibt sich ein effektiv betriebener Gesamtprozess, der in der Stoffbilanz als Produkte nur gebrannten Kalk, Dolomit, Magnesia oder Zement, Methan oder Methanol und Sauerstoff erzeugt und damit frei von Kohlenstoffdioxidemissionen ist, da das aus Verbrennungs- und Calcinierungsprozessen emittierte Kohlenstoffdioxid vollständig verwendet werden kann z. B. zur Methan/Methanol-Synthese und/oder zur Kühlung und/oder zur Brennstoffgemischbeimengung.

Besonders effektiv lässt sich das erfindungsgemäße Verfahren mit überschüssigem Strom aus Windkraft und Photovoltaik durchführen, indem klimaneutrales Methan/Methanol erzeugt wird, das, sofern es im Überschuss vorliegt, in vorhandene Erdgasnetze eingespeist werden kann. Hervorzuheben ist auch die Nutzung des Elektrolyse-Sauerstoffs als Oxidationsmittel im thermischen Prozess beim Kalk-, Dolomit-, Magnesia- oder Zementbrennen. Für die Methanisierung dient beim erfindungsgemäßen Verfahren ein nahezu reines Kohlenstoffdioxid-Abgas. Insgesamt gelingt mit der Erfindung eine emissionsfreie Kalk- oder Dolomit- oder Magnesia- oder Zementproduktion.

Die Erfindung schafft somit eine Kalk-, Dolomit-, Magnesia- oder Zementbrennanlage bzw. eine Schachtofenanlage, die ohne CO₂-Ausstoß in die Atmosphäre betrieben werden kann.

Es liegt im Rahmen der Erfindung, zusätzlich zum werksanlagenseitig erzeugten Sauerstoff und/oder Wasserstoff und/oder Methan oder Methanol entsprechende von Dritten bezogene Erzeugnisse zu verwenden.

Die Erfindung betrifft im Wesentlichen ein Verfahren zum Betreiben eines gebrannten Kalk, Dolomit, Magnesia oder Zement aus karbonathaltigen Rohstoffen erzeugenden Schachtofens, insbesondere ein Verfahren zum Betreiben eines Schachtofens zur Erzeugung von gebranntem Kalk oder Zement, wobei der Schachtofen zumindest eine obere Rohstoff-Einlaufzone, mindestens eine mittlere Brennzone und mindestens eine untere Brenngut-Auslaufzone aufweist,
gekennzeichnet durch die folgenden Maßnahmen:
a) Verbrennen eines Brennstoffgemisches aus einem kohlenstoffhaltigen Brennstoff aus Methan oder Methanol und industriell erzeugtem technischem Sauerstoff in der Brennzone des Schachtofens und Calcinieren des karbonathaltigen Rohstoffs in der Brennzone und oberhalb davon, wobei zusätzlich in die Brennzone und/oder die Auslaufzone des Schachtofens Kohlenstoffdioxid jeweils als Substitut der nach dem Stand der Technik üblicherweise verwendeten stickstoffhaltigen, atmosphärischen Luft, eingeleitet wird
b) Entnahme eines wenigstens 80, vorzugsweise über 90 Vol.-% Kohlenstoffdioxid enthaltenden Abgases, resultierend aus der Verbrennung des Brennstoffs, des Methans oder Methanols, mit Sauerstoff, dem zugeführten Kohlenstoffdioxid und der thermischen Zersetzung des jeweiligen carbonathaltigen Rohstoffs an der Einlauföffnung der Rohstoff-Einlaufzone und Einleiten des Abgases in einen Abgasspeicher, wobei das gemäß Merkmal a) zugeführte Kohlenstoffdioxid dem Kohlenstoffdioxid enthaltenden Abgasspeicher entnommen wurde
c) Erzeugen von Methan oder Methanol in einem Methanisierungs- oder Methanolisierungsreaktor aus aus dem Abgasspeicher dem Reaktor zugeführten Kohlendioxid und dem Reaktor zugeführten Wasserstoff und Einleitung des Methans oder Methanols in einen Methan- oder Methanolspeicher
d) Entnahme von Methan oder Methanol aus dem Methan- oder Methanolspeicher und Zuführen des Methans oder Methanols als Brennstoff zur Brennzone des Schachtofens
e) Erzeugung von Wasserstoff und Sauerstoff aus Wasser unter Verwendung von dem Methanisierungs- oder Methanolisierungsreaktor entnommenen Wasser durch Elektrolyse in einer mit elektrischem Strom unter Verwendung von einer werksanlageneigenen, mit aus dem Methan- oder Methanolspeicher entnommenen Methan oder Methanol betriebenen Stromerzeugungsanlage entnommenem elektrischen Strom betriebenen Elektrolyseanlage und Speichern des Wasserstoffs in einem Wasserstoffspeicher und des Sauerstoffs in einem Sauerstoffspeicher
f) Zuführen von Wasserstoff in den Methan- oder Methanolreaktor für die Durchführung der Methanisierung oder Methanolisierung durch Entnahme von Wasserstoff aus dem Wasserstoffspeicher
g) Zuführen von Sauerstoff in die Brennzone und/oder die Auslaufzone des Schachtofens durch Entnahme von Sauerstoff aus dem Sauerstoffspeicher.

Es liegt im Rahmen der Erfindung, dieses erfindungsgemäße Verfahren zu kombinieren mit mindestens einem Merkmal der folgenden Merkmale:
- für die Elektrolyse wird zusätzlich aus dem öffentlichen Stromnetz entnommener elektrischer Strom verwendet,
- für die Elektrolyse wird zusätzlich überschüssiger elektrischer Strom aus erneuerbaren Energien verwendet,
- für die Elektrolyse wird zusätzlich aus dem öffentlichen Wassernetz entnommenes Wasser verwendet,
- zusätzlich zur Verbrennung wird aus dem Erdgasnetz bezogenes Methan oder Methanol verwendet,
- zusätzlich zum aus der Elektrolyse stammenden Sauerstoff wird zur Verbrennung des Brennstoffs von Dritten bezogener technischer Sauerstoff verwendet,
- zusätzlich für die Durchführung der Methanisierung oder Methanolisierung wird von Dritten bezogener Wasserstoff verwendet.

Die Erfindung betrifft hauptsächlich auch eine Schachtofen-Werksanlage zur Erzeugung von gebranntem Kalk, Dolomit, Magnesia, vorzugsweise zur Erzeugung von Kalk oder Zement, insbesondere eingerichtet zur Durchführung des erfindungsgemäßen Verfahrens, die gekennzeichnet ist durch eine Schachtofeneinrichtung mit mindestens einem mindestens eine Rohstoff-Einlaufzone, mindestens eine mittlere Brennzone und mindestens eine untere Brenngut-Auslaufzone aufweisenden Schachtofen 2 in Kombination mit mindestens folgenden zugeordneten Einrichtungen:
- mindestens einer Abgasleitung 3 vom Schachtofeneinlauf zu einem Abgasspeicher 4
- mindestens einem Abgasspeicher 4
- mindestens einem Methanisierungs- oder Methanolisierungsreaktor 8
- mindestens einem Methan- oder Methanolspeicher 10
- mindestens einer mit Wasser und elektrischem Strom betriebenen Wasserelektrolyseanlage 15
- mindestens einer Abgasleitung 5, 6 vom Abgasspeicher 4 zur Brennzone und/oder Auslaufzone des Schachtofens 2.
- mindestens einen der Elektrolyseanlage 15 zugeordneten Wasserstoffspeicher 26 mit mindestens einer Wasserstoffzuleitung 27 zum Methanisierungs- oder Methanolisierungsreaktor 8
- mindestens einen der Elektrolyseanlage 15 zugeordneten Sauerstoffspeicher 20 mit mindestens einer Sauerstoffzuleitung 21/22 zur Brennzone und/oder Auslaufzone des Schachtofens 2
- mindestens eine Methan- oder Methanolzuführung 28 vom Methan- oder Methanolspeicher 10 zur Brennzone des Schachtofens 2
- eine elektrische Stromerzeugungsanlage 16, die elektrischen Strom an die Elektrolyseanlage liefern kann
- eine Stromerzeugungsanlage 16, die so ausgebildet und eingerichtet ist, dass sie mit aus dem Methan- oder Methanolspeicher 8 entnommenen Methan oder Methanol betreibbar ist

Es liegt im Rahmen der Erfindung, diese erfindungsgemäße Werksanlage zu kombinieren mit mindestens einem Merkmal der folgenden Merkmale, die gekennzeichnet sind durch:
- eine Wasserelektrolyseanlage 15, die über mindestens eine Stromzuleitung 14 aus dem öffentlichen Netz 13 und mindestens eine von der Stromerzeugungsanlage 16 kommende Stromzuleitung 18 verfügt
- eine Methan oder Methanol vom Methan- oder Methanolspeicher 10 ins Erdgasnetz führende Gas- bzw. Flüssigkeitsleitung 29.

Allgemein betrifft die Erfindung somit ein Verfahren zur gekoppelten Herstellung von organischen Kohlenwasserstoffen und anorganischen Oxiden, wobei die anorganischen Oxide durch thermische Zersetzung von anorganischen Carbonaten und Silikaten unter Zuführung eines Sauerstoff-Kohlenstoffdioxid-Brennstoff-Gemisches gebildet werden und der dabei in Form von Kohlenstoffdioxid abgespaltene Kohlenstoff zumindest teilweise als Basiselement für die Herstellung der organischen Kohlenwasserstoffe dient, wobei dessen molekularer Bindungspartner Sauerstoff durch elektrochemisch gewonnenen Wasserstoff unter Bildung fühlbarer Wärmeenergie zumindest teilweise chemisch ausgetauscht wird, und die dabei entstehenden Kohlenwasserstoffe zumindest teilweise durch Oxidation mit sauerstoffhaltigem Gas umgesetzt werden und dabei die notwendige thermische Energie für die thermische Zersetzung der anorganischen Carbonate und Silikate zumindest teilweise bereitgestellt wird.

Ein besonderer Vorteil der Erfindung liegt auch darin begründet, dass die erfindungsgemäß produzierten Erzeugnisse Methan/Methanol, Wasserstoff oder Sauerstoff hochpreisig teilweise verkauft werden können, insbesondere wenn entsprechende von Dritten angebotene und bezogene Erzeugnisse billiger sind.

## Patentansprüche

1. Verfahren zum Betreiben eines gebrannten Kalk, Dolomit, Magnesia oder Zement aus karbonathaltigen Rohstoffen erzeugenden Schachtofens (2), insbesondere ein Verfahren zum Betreiben eines Schachtofens (2) zur Erzeugung von gebranntem Kalk oder Zement, wobei der Schachtofen (2) zumindest eine obere Rohstoff-Einlaufzone, mindestens eine mittlere Brennzone und mindestens eine untere Brenngut-Auslaufzone aufweist, **gekennzeichnet durch** die folgenden Maßnahmen:
a) Verbrennen eines Brennstoffgemisches aus einem kohlenstoffhaltigen Brennstoff aus Methan oder Methanol und industriell erzeugtem technischem Sauerstoff in der Brennzone des Schachtofens (2) und Calcinieren des karbonathaltigen Rohstoffs in der Brennzone und oberhalb davon, wobei zusätzlich in die Brennzone und/oder die Auslaufzone des Schachtofens (2) Kohlenstoffdioxid jeweils als Substitut der nach dem Stand der Technik üblicherweise verwendeten stickstoffhaltigen, atmosphärischen Luft, eingeleitet wird
b) Entnahme eines wenigstens 80, vorzugsweise über 90 Vol.-% Kohlenstoffdioxid enthaltenden Abgases, resultierend aus der Verbrennung des Brennstoffgemisches, dem zugeführten Kohlenstoffdioxid und der thermischen Zersetzung des jeweiligen carbonathaltigen Rohstoffs, an der Einlauföffnung der Rohstoff-Einlaufzone und Einleiten des Abgases in einen Abgasspeicher (4), wobei das gemäß Merkmal a) zugeführte Kohlenstoffdioxid dem Kohlenstoffdioxid enthaltenden Abgasspeicher (4) entnommen wurde
c) Erzeugen von Methan oder Methanol in einem Methanisierungs- oder Methanolisierungsreaktor (8) aus aus dem Abgasspeicher (4) dem Reaktor (8) zugeführten Kohlenstoffdioxid und dem Reaktor (8) zugeführten Wasserstoff und Einleitung des Methans oder Methanols in einen Methan- oder Methanolspeicher (10)
d) Entnahme von Methan oder Methanol aus dem Methan- oder Methanolspeicher (10) und Zuführen des Methans oder Methanols als Brennstoff zur Brennzone des Schachtofens (2)
e) Erzeugung von Wasserstoff und Sauerstoff aus Wasser unter Verwendung von dem Methanisierungs- oder Methanolisierungsreaktor (8) entnommenem Wasser **durch** Elektrolyse in einer mit elektrischem Strom unter Verwendung von einer werksanlageneigenen, mit aus dem Methan- oder Methanolspeicher (10) entnommenen Methan oder Methanol betriebenen Stromerzeugungsanlage (16) entnommenem elektrischen Strom betriebenen Elektrolyseanlage (15) und Speichern des Wasserstoffs in einem Wasserstoffspeicher (26) und des Sauerstoffs in einem Sauerstoffspeicher (20)
f) Zuführen von Wasserstoff in den Methanisierungs- oder Methanolisierungsreaktor (8) für die Durchführung der Methanisierung oder Methanolisierung aus dem Wasserstoffspeicher (26)
g) Zuführen von Sauerstoff in die Brennzone und/oder die Auslaufzone des Schachtofens (2) aus dem Sauerstoffspeicher (20)

2. Verfahren nach Anspruch 1,
**dadurch gekennzeichnet,dass**
zusätzlich für die Elektrolyse überschüssiger elektrischer Strom aus erneuerbaren Energien verwendet wird.

3. Verfahren nach Anspruch 1 und/oder 2,
**dadurch gekennzeichnet,dass**
zusätzlich für die Elektrolyse dem öffentlichen Wassernetz (12) entnommenes Wasser verwendet wird.

4. Verfahren nach einem oder mehreren der Ansprüche 1 bis 3,
**dadurch gekennzeichnet,dass**
zusätzlich zur Verbrennung aus dem Erdgasnetz bezogenes Methan oder Methanol verwendet wird.

5. Verfahren nach einem oder mehreren der Ansprüche 1 bis 4
**dadurch gekennzeichnet, dass**
zusätzlich zum aus der Elektrolyse stammenden Sauerstoff zur Verbrennung des Brennstoffs von Dritten bezogener technischer Sauerstoff verwendet wird.

6. Schachtofen-Werksanlage zur Erzeugung von gebranntem Kalk, Dolomit, Magnesia, vorzugsweise zur Erzeugung von Kalk oder Zement, insbesondere eingerichtet zur Durchführung des Verfahrens nach einem oder mehreren der Ansprüche 1 bis 5,
**gekennzeichnet** t durch eine Schachtofeneinrichtung mit mindestens einem mindestens eine Rohstoff-Einlaufzone, mindestens eine mittlere Brennzone und mindestens eine untere Brenngut-Auslaufzone aufweisenden Schachtofen (2) in Kombination mit mindestens folgenden zugeordneten Einrichtungen:
- mindestens einer Abgasleitung (3) vom Schachtofeneinlauf zu einem Abgasspeicher (4)
- mindestens einem Abgasspeicher (4)
- mindestens einem Methanisierungs- oder Methanolisierungsreaktor (8)
- mindestens einem Methan- oder Methanolspeicher (10)
- mindestens einer mit Wasser und elektrischem Strom betriebenen Wasserelektrolyseanlage (15)
- mindestens einer Abgasleitung (5, 6) vom Abgasspeicher (4) zur Brennzone und/oder Auslaufzone des Schachtofens (2)
- mindestens einem der Elektrolyseanlage (15) zugeordneten Wasserstoffspeicher (26) mit mindestens einer Wasserstoffzuleitung (27) zum Methanisierungs- oder Methanolisierungsreaktor (8)
- mindestens einem der Elektrolyseanlage (15) zugeordneten Sauerstoffspeicher (20) mit mindestens einer Sauerstoffzuleitung (21/22) zur Brennzone und/oder Auslaufzone des Schachtofens (2)
- mindestens einer Methan- oder Methanolzuführung (28) vom Methan- oder Methanolspeicher (10) zur Brennzone des Schachtofens (2)
- einer elektrischen Stromerzeugungsanlage (16), die elektrischen Strom an die Wasserelektrolyseanlage liefern kann,
wobei die Stromerzeugungsanlage (16) so ausgebildet und eingerichtet ist, dass sie mit aus dem Methan- oder Methanolspeicher (8) entnommenen Methan oder Methanol betreibbar ist.

7. Werksanlage nach Anspruch 6,
**dadurch gekennzeichnet,dass**
die Wasserelektrolyseanlage (15) über mindestens eine Stromzuleitung (14) aus dem öffentlichen Netz (13) und mindestens eine von der Stromerzeugungsanlage (16) kommenden Stromzuleitung (18) verfügt.

8. Werksanlage nach einem der Ansprüche 6 oder 7,
**gekennzeichnet** t durch eine Methan oder Methanol vom Methan- oder Methanolspeicher (10) ins Erdgasnetz führende Gas- bzw. Flüssigkeitsleitung (29).

## Claims

1. Method for operating a shaft furnace (2) producing burnt lime, dolomite, magnesia or cement from carbonate-containing raw materials, in particular a method for operating a shaft furnace (2) for producing burnt lime or cement, the shaft furnace (2) having at least one upper raw material inlet zone, at least one central burning zone and at least one lower burnt material outlet zone, **characterised by** the following measures:
a) burning a fuel mixture of a carbon-containing fuel made of methane or methanol and industrially produced tonnage oxygen in the burning zone of the shaft furnace (2) and calcining the carbonate-containing raw material in the burning zone and thereabove, carbon dioxide being additionally introduced into the burning zone and/or the outlet zone of the shaft furnace (2) in each case as a substitute for the nitrogen-containing, atmospheric air customarily used according to the prior art
b) removing a waste gas containing at least 80, preferably more than 90, percent by volume of carbon dioxide and resulting from the burning of the fuel mixture, the supplied carbon dioxide and the thermal decomposition of the respective carbonate-containing raw material, at the inlet opening of the raw material inlet zone and leading the waste gas into a waste gas store (4), the carbon dioxide supplied according to feature a) having been withdrawn from the waste gas store (4) containing the carbon dioxide
c) producing methane or methanol in a methanation or methanolation reactor (8) from carbon dioxide supplied to the reactor (8) from the waste gas store (4) and hydrogen supplied to the reactor (8) and leading the methane or methanol into a methane or methanol store (10)
d) withdrawing methane or methanol from the methane or methanol store (10) and supplying the methane or methanol as fuel to the burning zone of the shaft furnace (2)
e) producing hydrogen and oxygen from water, using water withdrawn from the methanation or methanolation reactor (8), by electrolysis in an electrolysis system (15) operated by electric current drawn from a plant'sown current-generating plant (16) operated by methane or methanol withdrawn from the methane or methanol store (10) and storing the hydrogen in a hydrogen store (26) and the oxygen in an oxygen store (20)
f) feeding hydrogen into the methanation or methanolation reactor (8) from the hydrogen store (26) for carrying out the methanation or methanolation
g) feeding oxygen into the burning zone and/or the outlet zone of the shaft furnace (2) from the oxygen store (20).

2. Method according to Claim 1,
**characterised in that**
surplus electric current from renewable energies is additionally used for the electrolysis.

3. Method according to Claim 1 and/or 2,
**characterised in that**
water drawn from the public water network (12) is additionally used for the electrolysis.

4. Method according to one or more of Claims 1 to 3,
**characterised in that**
methane or methanol obtained from the natural gas network is additionally used for the burning.

5. Method according to one or more of Claims 1 to 4,
**characterised in that**
tonnage oxygen obtained from third parties is used for the combustion of the fuel in addition to the oxygen coming from the electrolysis.

6. Shaft furnace plant for producing burnt lime, dolomite, magnesia, preferably for producing lime or cement, in particular adapted for carrying out the method according to one or more of Claims 1 to 5,
**characterised by** a shaft furnace installation having at least one shaft furnace (2) comprising at least one raw material inlet zone, at least one central burning zone and at least one lower burnt material outlet zone, in combination with at least the following associated apparatus:
- at least one waste gas line (3) from the shaft furnace inlet to a waste gas store (4)
- at least one waste gas store (4)
- at least one methanation or methanolation reactor (8)
- at least one methane or methanol store (10)
- at least one water electrolysis system (15) operated by water and electric current
- at least one waste gas line (5, 6) from the waste gas store (4) to the burning zone and/or outlet zone of the shaft furnace (2)
- at least one hydrogen store (26) associated with the electrolysis system (15) and having at least one hydrogen supply line (27) to the methanation or methanolation reactor (8)
- at least one oxygen store (20) associated with the electrolysis system (15) and having at least one oxygen supply line (21/22) to the burning zone and/or outlet zone of the shaft furnace (2)
- at least one methane or methanol supply (28) from the methane or methanol store (10) to the burning zone of the shaft furnace (2)
- an electric current-generating plant (16) which can supply electric current to the water electrolysis system,
the current-generating plant (16) being configured and adapted such that it can be operated by methane or methanol withdrawn from the methane or methanol store (8).

7. Plant according to Claim 6,
**characterised in that**
the water electrolysis system (15) has at least one current supply line (14) from the public network (13) and at least one current supply line (18) coming from the current-generating plant (16).

8. Plant according to one of Claims 6 or 7,
**characterised by** a gas or liquid line (29) leading methane or methanol from the methane or methanol store (10) into the natural gas network.

## Revendications

1. Procédé servant à faire fonctionner un four droit (2) produisant de la chaux vive, de la dolomite, de la magnésie ou du ciment à partir de matières premières contenant du carbonate, en particulier procédé servant à faire fonctionner un four droit (2) servant à produire de la chaux vive ou du ciment, dans lequel le four droit (2) présente au moins une zone supérieure d'admission de matière première, au moins une zone centrale de combustion et au moins une zone inférieure d'évacuation de produit combustible, **caractérisé par** les mesures qui suivent consistant à :
a) incinérer un mélange de combustibles composé d'un combustible contenant du carbone, composé de méthane ou de méthanol, et d'un oxygène technique produit industriellement, dans la zone de combustion du four droit (2) et calciner la matière première contenant du carbonate dans la zone de combustion et au-dessus de cette dernière, dans lequel, en supplément, du dioxyde de carbone est introduit, respectivement en tant que substitut de l'air atmosphérique contenant de l'azote, utilisé généralement selon l'état de la technique dans la zone de combustion et/ou dans la zone d'évacuation du four droit (2)
b) prélever des gaz d'échappement contenant au moins 80 % en volume, de préférence plus de 90 % en volume de dioxyde de carbone, issus de l'incinération du mélange de combustibles, du dioxyde de carbone amené et de la décomposition thermique de la matière première respective contenant du carbonate, au niveau de l'ouverture d'admission de la zone d'admission de matière première et introduire les gaz d'échappement dans un réservoir de stockage de gaz d'échappement (4), dans lequel le dioxyde de carbone amené selon la caractéristique a) a été prélevé du réservoir de stockage de gaz d'échappement (4) contenant du dioxyde de carbone
c) produire du méthane ou du méthanol dans un réacteur de méthanisation ou de méthanolisation (8) à partir du dioxyde de carbone amené au réacteur (8) depuis le réservoir de stockage de gaz d'échappement (4) et à partir de l'hydrogène amené au réacteur (8) et introduire le méthané ou le méthanol dans un réservoir de stockage de méthane ou de méthanol (10)
d) prélever du méthane ou du méthanol du réservoir de stockage de méthane ou de méthanol (10) et amener le méthane ou le méthanol en tant que combustible jusqu'à la zone de combustion du four droit (2)
e) produire de l'hydrogène et de l'oxygène à partir d'eau en utilisant l'eau prélevée du réacteur de méthanisation ou de méthanolisation (8) par électrolyse en utilisant une unité d'électrolyse (15) propre à l'usine fonctionnant avec un courant électrique prélevé d'une unité de production de courant (16) fonctionnant avec du méthane ou du méthanol prélevé du réservoir de stockage de méthane ou de méthanol (10) et stocker l'hydrogène dans un réservoir de stockage d'hydrogène (26) et l'oxygène dans un réservoir de stockage d'oxygène (20)
f) amener de l'hydrogène provenant du réservoir de stockage d'hydrogène (26) dans le réacteur de méthanisation ou de méthanolisation (8) en vue de la mise en oeuvre de la méthanisation ou de la méthanolisation
g) amener de l'oxygène provenant du réservoir de stockage d'oxygène (20) dans la zone de combustion et/ou dans la zone d'évacuation du four droit (2).

2. Procédé selon la revendication 1,
**caractérisé en ce que**
en supplément, on utilise, pour l'électrolyse, un excédent de courant électrique issu d'énergies renouvelables.

3. Procédé selon la revendication 1 et/ou 2,
**caractérisé en ce que**
en supplément, on utilise, pour l'électrolyse, de l'eau prélevée du réseau d'eau public (12).

4. Procédé selon l'une quelconque ou plusieurs des revendications 1 à 3,
**caractérisé en ce que**
en supplément, on utilise, aux fins de l'incinération, du méthane ou du méthanol provenant du réseau de gaz naturel.

5. Procédé selon l'une quelconque ou plusieurs des revendications 1 à 4,
**caractérisé en ce que**
en supplément de l'oxygène issu de l'électrolyse, on utilise, aux fins de l'incinération du combustible, un oxygène technique fourni par des tiers.

6. Unité d'usine à four droit servant à produire de la chaux vive, de la dolomite, de la magnésie, de préférence servant à produire de la chaux ou du ciment mise au point en particulier afin de mettre en oeuvre le procédé selon l'une quelconque ou plusieurs des revendications 1 à 5,
**caractérisée par** un système à four droit comprenant au moins un four droit (2) présentant au moins une zone d'admission de matière première, au moins une zone de combustion centrale et au moins une zone d'évacuation de produit combustible inférieure, en combinaison avec au moins les systèmes associés qui suivent :
- au moins une conduite de gaz d'échappement (3) allant de l'admission du four droit à un réservoir de stockage de gaz d'échappement (4)
- au moins un réservoir de stockage de gaz d'échappement (4)
- au moins un réacteur de méthanisation ou de méthanolisation (8)
- au moins un réservoir de stockage de méthane ou de méthanol (10)
- au moins une unité d'électrolyse d'eau (15) fonctionnant avec de l'eau et du courant électrique
- au moins une conduite de gaz d'échappement (5, 6) allant du réservoir de stockage de gaz d'échappement (4) à la zone de combustion et/ou à la zone d'évacuation du four droit (2)
- au moins un réservoir de stockage d'hydrogène (26) associé à l'unité d'électrolyse (15), comprenant au moins une conduite d'alimentation en hydrogène (27) menant au réacteur de méthanisation ou de méthanolisation (8)
- au moins un réservoir de stockage d'oxygène (20) associé à l'unité d'électrolyse (15), comprenant au moins une conduite d'alimentation en oxygène (21/22) menant à la zone de combustion et/ou à la zone d'évacuation du four droit (2)
- au moins une alimentation en méthane ou en méthanol (28) allant du réservoir de stockage de méthane ou de méthanol (10) vers la zone de combustion du four droit (2)
- une unité de production de courant électrique (16), qui peut fournir du courant électrique à l'unité d'électrolyse d'eau,
dans laquelle l'unité de production de courant (16) est réalisée et mise au point de telle sorte qu'elle peut fonctionner avec du méthane ou du méthanol prélevé du réservoir de stockage de méthane ou de méthanol (8).

7. Unité d'usine selon la revendication 6,
**caractérisée en ce que**
l'unité d'électrolyse d'eau (15) dispose d'au moins une alimentation en courant (14) provenant du réseau public (13) et d'au moins une conduite d'alimentation en courant (18) venant de l'unité de production de courant (16).

8. Unité d'usine selon l'une quelconque des revendications 6 ou 7,
**caractérisée par** une conduite de gaz ou de liquide (29) acheminant du méthane ou du méthanol provenant du réservoir de stockage de méthane ou de méthanol (10) dans le réseau de gaz naturel.
